# EUROPEAN PATENT SPECIFICATION

(11) **EP 2 117 342 B1**
(45) Date of publication and mention of the grant of the patent: **20.04.2016**
(21) Application number: 08712666.0
(22) Date of filing: 08.02.2008
(51) Int. Cl.: A23K 50/80, A23K 20/147, A61P 31/12, A23K 20/158, A23K 20/153, A61P 1/14

(54) **FEED COMPOSITION FOR SALMONIDS AND USES THEREOF**
FUTTERMITTELZUSAMMENSETZUNG UND IHRE VERWENDUNGEN
COMPOSITION ALIMENTAIRE ET SES UTILISATIONS

(30) Priority: 08.02.2007 NO 20070767
(43) Date of publication of application: 18.11.2009
(73) Proprietor: Ewos Innovation AS, 4335 Dirdal (NO)
(72) Inventor: VIKE, Siri, N-5259 Hjellestad (NO); MYHR, Egil, N-5043 Bergen (NO); WADSWORTH, Simon, N-1383 Asker (NO); VECINO, José, L., Gonzalez, N-4013 Stavanger (NO); EL-MOWAFI, Adel, N-4308 Sandnes (NO)
(74) Representative: Hindenes, Jan-Ove
(86) International application number: PCT/NO2008/000050
(87) International publication number: WO 2008/097103

(56) References cited:
- WO-A-01/06868
- WO-A-98/19560
- WO-A-2006/009464
- ENCARNACAO ET AL: "Diet energy source affects lysine utilization for protein deposition in rainbow trout (Oncorhynchus mykiss)" AQUACULTURE, ELSEVIER, vol. 261, no. 4, 28 November 2006 (2006-11-28), pages 1371-1381, XP005781662 ISSN: 0044-8486
- KROGDAHL, A., BAKKE-MCKELLEP, A.M., AND BAEVERFJORD, G.: "Effects of graded levels of standard soybean meal on intestinal structure, mucosal enzyme activities, and pancreatic response in Atlantic salmon (Salmo salar L.)" AQUACULTURE NUTRITION, vol. 9, 2003, pages 361-371, XP002480387 GBBLACKWELL SCIENCE, OXFORD
- RAYNARD, R.S, MCVICAR, A.H., BELL, J.G., YOUNGSON, A., KNOX, D., AND FRASER, C.O.: "Nutritional aspects of pancreas disease of atlantic salmon: the effects of dietary vitamin E and polyunsaturated fatty acids" COMPARATIVE BIOCHEMISTRY AND PHYSIOLOGY. PART A. COMPARATIVE PHYSIOLOGY., vol. 98a, 1991, pages 125-131, XP002480388 ELSEVIER SCIENCE LTD.
- REFSTIE, S., BAKKE-MCKELLEP, A.M., PENN, M.H., SUNDBY, A., SHEARER, K. D., KROGDAHL, A.: "Capacity for digestive hydrolysis and amino acid absorption in Atlantic salmon (Salmo salar) fed diets with soybean meal or inulin with or without the addition of antibiotics" Aquaculture, vol. 261, 2006, pages 392-406, XP005848606 Elsevier
- DAMSGARD B ET AL: "Effects of feeding regime on susceptibility of Atlantic salmon (Salmo salar) to cold water vibriosis" AQUACULTURE, ELSEVIER, vol. 239, no. 1-4, 30 September 2004 (2004-09-30), pages 37-46, XP004549086 ISSN: 0044-8486

## Description

### FIELD OF THE INVENTION

The present invention concerns a feed composition for use in alleviation, faster recovery and/or treatment of salmonids with Pancreas disease. The composition is suitable in reducing of the negative effects of an outbreak of pancreas disease in salmonids, meaning that the composition functions as a clinical diet during a period of a dysfunctional pancreas in the diseased fish.

### BACKGROUND OF THE INVENTION

Pancreas disease and other virus infections in fish are a serious problem in aquaculture of many aquatic species including salmonids such as the Atlantic salmon *Salmo salar.* These infections do not only frequently cause enormous economical losses in aquaculture production, they are also an important issue of animal well-fare. Awareness of animal well-fare by consumers, producers and national authorities has increased tremendously in the past, also including the aquaculture sector. Thus, focus on the prevention, efficient treatment and/or reduction of mortality and agony connected to disease outbreaks such as Pancreas disease and other virus infections is of great importance.

Disease influences the fishes feeding behaviour. Feeding diseased salmonid fish during a clinical outbreak of Pancreas disease is a significant challenge, outbreaks lasting from 3 to 6 months. Dietary amelioration is dependant on a correct and early PD diagnosis and an early deployment of a PD diet. Effective site monitoring of feeding behaviour and mortalities, using monitoring system is an essential tool in PD disease management.

From the mid nineties, it has been known that the pancreas disease (PD) in Atlantic salmon (*salmo salar*) and Rainbow trout (*onchorynchus mykiss*) is caused by a virus, called Salmonid Alpha Virus (SAV) who belongs to the Togaviridae family. Typical Pancreatic disease-symptoms in fish are a reduced appetite, as well as an increased mortality. The pathology of the disease is very similar to other infections, but small bleedings can be registered in the adipose tissue as well as some clear liquid in the abdominal cavity. The liver can be somewhat swollen with a fibrin layer. Chronically disordered fish are stripped down and possess little fat in the abdominal cavity. The atrophy of the pancreas and inflammation and necrosis of the heart and skeletal muscle are the most important organ alterations.

The dietary effect of high and low amounts of vitamin E and polyunsaturated fatty acids have been tested on the susceptibility for pancreatic disease in salmon (Raynard, R. S.; McVicar, A. H.; Bell, J. G.; Youngson, A.; Knox, D.; Fraser, C.O. 1991. Nutritional aspects of pancreatic disease of Atlantic salmon: the effects of dietary vitamin E and polyunsaturated fatty acids. Comparative Biochemistry and Physiology. Part A. Comparatice Physiology. Vol. 98a, p. 125-131).

Experimental diets, dietary compositions, and feed having a high protein to lipid ratio, e.g. a protein:lipid ratio higher than 1 are principally known (Encarnacao, P., de Lange, C., Bureau, D.P. 2006. Diet energy source affects lysine utilization for protein deposition in rainbow trout (Oncorhynchus mykiss). Aquaculture 261, p. 1371-1381; Krogdahl, A., Bakke-McKellep, A. M., Baeverfjord, G. 2003. Effects of graded levels of standard soybean meal on intestinal structure , mucosal enzyme activities, and pancreatic response in Atlantic salmon (Salmo salar L.). Aquaculture Nutrition. Vol. 9, p. 361-371; WO 2006/009464 A; WO 01/06868 AA; WO 98/19560 A; Refstie, S., Bakke-McKellep, A.M., Penn, M.H., Sundby, A., Shearer, K. D., Kroghdahl, A. Capacity for digestive hydrolysis and amino acids absorption in Atlantic Salmon (Salmo salar) fed diets with soybean meal or inulin with or without the addition of antibiotics. Aquaculture, Vol. 261, p. 392-406), however no effects on pancreatic disease in fish have been described. Efforts to develop a vaccine have been without significant success so far.

The present invention aims thus at providing a feed composition which can effectively be used reduce negative effects of Pancreas disease infection in salmonids.

Moreover, the present invention aims at providing a feed composition, which effectuates that fish is able to absorb/ingest adequate nutrition during a critical phase causing that it survives and that vital organs regenerate.

Furthermore, the present invention aims at providing a feed composition, which effectuates that the fish recovers its appetite and at the same time enables the fish to cope with the challenge of a virus infection or a Pancreas disease outbreak, which thereby reduces the mortality of the fish and which reduces the inflammatory reactions in actual affected tissues.

It is an aim of the present invention that the feed composition is administered to the fish as soon as the Pancreas disease outbreak has been discovered, and until the mortality caused by the outbreak has been finished.

### SUMMARY OF THE INVENTION

Surprisingly, it has been found that by amending the ratio and/or quantitative amounts between the main feed components protein and lipid, the fish is enabled to digest the feed even during a period when the fish is challenged by virus, or when it has developed Pancreas disease.

In a first aspect, the present invention relates to a us of a feed composition comprising conventional feed ingredients such as protein, lipid, ash, vitamins, minerals, carbohydrates, pigments, wherein the relation of protein to lipid is more than 1, based on weight, for use in alleviation, faster recovery, and/or treatment of salmonids infected with Pancreas disease, wherein the feed composition is provided from the registration of the first symptoms of the infection in fish or a fish population or from the detection of SAV virus in the population.

In a preferred embodiment, the relation of protein to lipid is in the range of 1 to 3, based on the weight, more preferable 1.5 to 2.5, based on weight, preferable about 2 based on weight.

A further embodiment has an amount of protein in relation to the total weight of the feed composition in the range of 30%to 70%, more preferable about 40% to 60%, and more preferable about 45% to 55%.

Other ranges described are from 10% to 40%, and from 20% to 30%.

In the context of the invention the amount of lipid in relation to the total weight of the feed composition can for example be less than 30%, less than 28%, less than 26%, or less than 25%.

It is an advantage that a portion of said protein is processed protein, such as in an amount of at least 10%, 20%, 25%, or 30%, based on the weight of the total protein.

Said processed protein can be selected from the group comprising hydrolyzed protein, water soluble protein and easily digestible protein.

Further embodiments comprise an immune stimulating and/or anti-inflammation compound.

Examples for immune stimulating and/or anti-inflammation compounds comprise glucan, such as beta-1,3/1,6-glucan or peptidoglycan.

In a further embodiment comprise said feed composition also semiessential nutrients such as nucleotides, nucleosides or both.

It is suggested that the feed composition can further comprise an active component, such as a pharmaceutical agent, such as an anti-viral component.

The feed composition according to the invention is useful for decreasing the mortality of salmonids infected with Pancreas disease.

Further, the feed composition according to the invention can be used for faster recovery of salmonids infected with Pancreas disease.

Preferable, the feed composition is provided to said salmonids from the registration of the first symptoms of an infection in fish or a fish population and/or in case of a risk for a Pancreas disease infection.

It is suggested to provide the feed composition the period from the discovery of a risk for a Pancreas disease infection up to 20 weeks after the determination of the disease.

It is also suggested that the feed composition can be provided about four weeks before the determination of the disease and until 15 weeks after the determination of the disease. It is an advantage that the composition is provided at least from about the determination of the disease and until 15 weeks after the determination of the disease, more preferable from about the determination of the disease and until 12 weeks after the determination of the disease.

The feed composition is suggested to also function as a vehicle or carrier for efficiently transport of pharmaceutical or nutritional components to the intestinal tract.

The composition can improve the capability to maintain or increase the biomass of said salmonids.

Without being bound to theoretical considerations, it is assumed that during this period of infection the fish has a reduced ability to digest a conventional feed, either due to the fact that the absorptive mechanisms are reduced or that the amount or activity of the digestive enzymes are reduced.

During testing of different feed compositions wherein a reduced amount of lipid in relation to protein has been used, it could be shown that this had a beneficial effect on the appetite as well as on the ability to survive and recover in connection to Pancreas disease.

Lipids are an important energy source in salmonids and the level of lipid in feed compositions has increased during the past decade to about 30-40 % for on-growing salmon. This has been shown to be beneficial in terms of growth. Commonly applied protein content in feed for salmonids is 30-40 %.

One problem connected to feeding of Pancreatic disease-infected fish is that the conventional fish feed is apparently badly absorbed by the gut, and that it passes through the intestinal tract without substantial effect probably since the necessary enzymes needed for hydrolyzing the feed ingredients are lacking. The feed composition of the present invention has shown an improved absorption in the digestive system of the fish and by that causing an improved utilization/absorption of the nutritional components of the feed composition and as well as of other additive substances such as immunostimulating and anti-inflammation materials.

Thus, a great effect can be obtained by developing a composition which can energetically be utilized by the fish even though its digestive system is significantly affected as result of a Pancreas disease infection, as well as that the effective feed composition can be used as a carrier for active components which one would like to administer to the fish. As exemplified, the later compounds could be immuno-stimulating agents and/or inflammation reducing agents and semi-essential nutrients, but they can also be compounds with nutritional or pharmaceutical effects.

It is assumed that by partly replacing of the protein component by processed proteins, such as peptides, e.g. in from of hydrolyzed material, the absorption of the feed composition and other active compounds is further increased.

The present invention also includes feeding of a combination of different feed compositions which overall do sum up to the same protein/lipid ratio when fed to the fish as defined in the claims of the present invention, for example by feeding a conventional feed with a protein/lipid ration of one or less than one in combination with a high protein diet.

It will be appreciated that features of the invention described in the foregoing can be combined in any combination without departing from the scope of the invention.

### DESCRIPTION OF THE DIAGRAMS

Embodiments of the invention will now be described, by way of examples, with reference to the following diagrams in which:
Figure 1 shows the accumulated mortality of salmon during feeding with test diet and a control diet of example 1.
Figure 2 shows changes in the biomass for fish fed the test diet or the control diet of example 1.
Figure 3 indicates the feeding rate, indicating the appetite of the fish fed the test diet or the control diet of example 1.

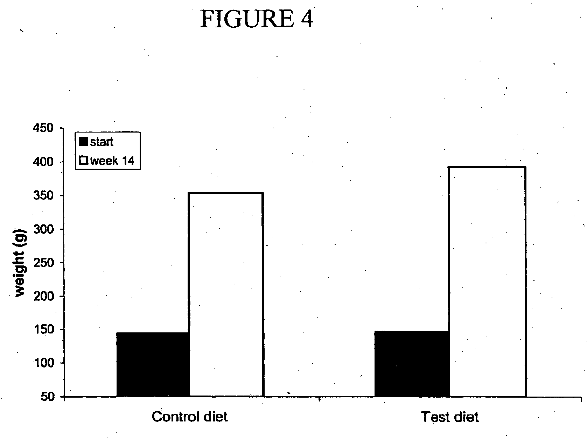

Figure 4 shows the accumulated growth of fish fed the test diet or the control diet of example 2.
Figure 5 shows pathological changes in Pancreas for fish the fed test diet or control diet of example 2.

### EXPERIMENTAL SECTION

Fish were fed *ad libitum,* meaning that the feed access was not a limiting factor.

### Feeding of fish with a Pancreatic disease-diagnose

### Example 1

The feed composition of the present invention has been tested in an aquaculture plant where Atlantic salmon had a diagnosed Pancreas disease infection in two of totally eight cages. Until the start of the experiment fish of all cages were treated comparable and received the same diet.

Three weeks after the diagnosis of Pancreas disease fish in all cages were fed two different diets, the test diet of the present invention and a control diet (table 1) for a period of 11 weeks.

**Table 1:**

| Feeding ingredients (% by weight) in the test diet and control diet. | | |
|---|---|---|
| | Test diet | Control diet |
| Total lipid content | 25 | 40 |
| Total protein content | 49 | 37 |
| Ash | 9 | 7 |
| Water | 9 | 7 |
| NFE | 11 | 9 |

Apart from the given ingredients, both diets contained conventional compounds included in fish feed such as antioxidants, vitamins, minerals, pigments, etc.

The main difference in the dietary composition of the test diet and the control diet was the protein/lipid ratio. The test diet comprised about 49 % protein and about 25 % lipid, based on the total weight of the feed composition, resulting in a protein to lipid ratio of about 2, while the control diet comprised about 37 % protein and about 40 % lipid, resulting in a protein/lipid ratio of about 0.9. The control diet thus corresponded to a typical commercial diet with a conventional dietary composition in terms of proteins and lipids as it is commonly used for on-growing salmon. In contrast, the test diet had an increased amount of protein and a reduced amount of lipid compared to commercially applied diets.

Feeding of the two different diets was carried out in eight cages with an initial biomass of about 60.000 kg per cage at the start of the experiment and an estimated average weight of about 770 gram per fish. Five cages received the control diet, while three cages received the test diet of the present invention. The cages for each of the treatments were chosen randomly. Fish were fed for about 11 weeks, when the experiment was finished. The mortality of fish, and increase of biomass in each cage was determined in defined intervals during the experimental period. Fish were fed according to their appetite (ad libitum). The feed ration provided to the fish in each of the cages was adapted individually for each cage on a daily basis based upon observations made with a camera placed at the bottom of each cage.

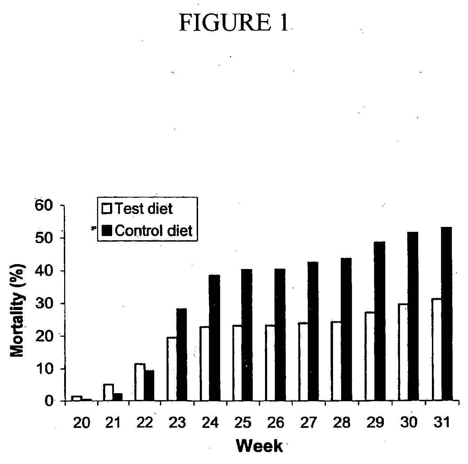

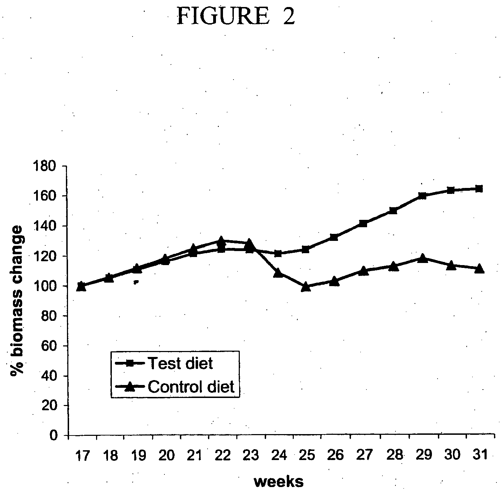

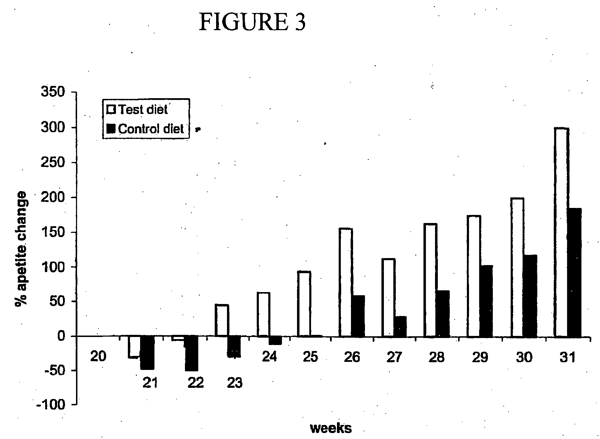

The accumulated mortality at the end of the feeding experiment was 53 % for fish fed the control diet while it was 31 % for the fish fed the test diet of the present invention (Figure 1). The relative increase in biomass in salmon fed the control diet was considerably lower compared to the biomass in salmon fed the test diet of the present invention increased during the same period (Figure 2). The growth pattern was also reflected in the actual feeding rate of the fish (Figure 3). Fish of both treatments had a reduced feeding intake during the first two weeks after feeding of the test diet and control diet was initiated. However, the reduction in fish fed the control diet was much higher than in fish fed the test diet of the present invention. Moreover, fish fed the test diet of the present invention had an increasing appetite already after 3 weeks after the start of the experiment, while fish of the control diet showed a clear delay in the increase of the feeding rate and had a much lower feeding rate throughout the whole experiment.

These results clearly demonstrate the beneficial effect of a high amount of protein in relation to lipids in terms of survival, growth and feeding rate of fish exposed to a Pancreas disease outbreak.

### Example 2:

A total of 1260 fish (70 fish per tank in 18 tanks) Atlantic salmon *Salmo salar,* start weight 100 g, seawater adapted, were fed for a period for 4 weeks at 2% body weight per day. The fish were maintained in ambient seawater tempertature at 100 fish per tank. After 4 weeks feeding control diet the fish were challenged with 0.2 ml of a dose predicted to kill 20% of the population (LD₄₀) of Salmon Alphavirus by intra-peritoneal (i.p.) injection. Fish were then fed test diets from day of challenge, for a further 14 weeks. The control diets and test diets were divided equally into the 9 tanks in both of the labs in random order. Mortalities were removed daily, weighed and assessed for Alphavirus. All fish individual weights and lengths were recorded at the start and end of the trial. Sampling of of the fish were done in week 3, 6, 9, 12 and 14 after infection.

**Table 2:**

| Feeding ingredients (% by weight) in the test diet and control diet. | | |
|---|---|---|
| | Test diet | Control diet |
| Total lipid content | 16 | 28 |
| Total protein content | 53 | 45 |
| Ash | 7.5 | 7.4 |
| Water | 7.3 | 6.2 |
| NFE | 16.2 | 13.4 |

Apart from the given ingredients, both diets contained conventional compounds included in fish feed such as antioxidants, vitamins, minerals, pigments, etc.

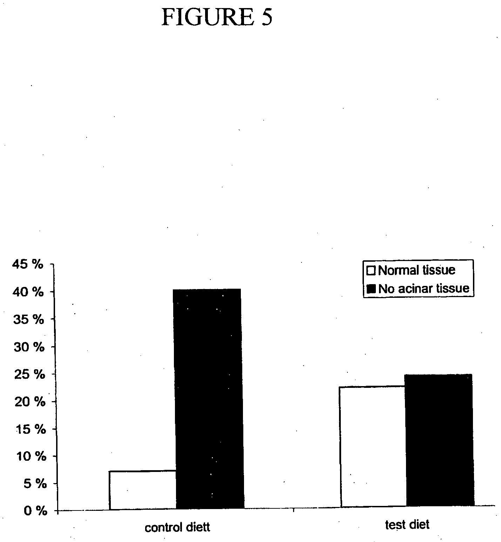

By histology we confirmed pathological changes in Pancreas, as shown in figure 5. The results are obtained 6 weeks after the fish were challenged with SAV. 22 % of the fish that received the test diet according to the invention has still a normal pancreas. In contrast, only 7 % of the fish that received the control diet has a normal pancreas. Further, 40 % of the fish that received the control diet has almost nothing acinar tissue, whereas only 24 % of the fish that received the test diet according to the invention has almost nothing acinar tissue. This clearly shows that the feed composition according to the present invention has an positive effect on the PD disease.

It will be appreciated that embodiments of the invention described in the foregoing can be modified without departing from the scope of the invention.

### Definitions

SAV: salmonid alpha virus belongs to the Togaviridae family and there are today three subtypes detected: SAV1, SAV2 and SAV3. The name Salmon is alpha virus is not yet approved in ICTV (international Committee on Taxonomy of Viruses), but it the common name used in the industry.

Salmonids: Fish belonging to the family Salmonidae. Representative examples are Atlantic salmon *(salmo salar),* Rainbow trout *(onchorynchus mykiss),* Coho salmon, *(Oncorhynchus kisutch)*

The term "enhancing fish welfare and viability" in this context means that the general condition of the fish in terms of health, stress- and disease-resistance is improved. As measurable indicators in the present invention may beside other factors serve improved survival rates and growth rates, improved appetite, low number of infected or seriously affected fish by pancreas disease as well as an improved ability to recover from an infection with pancreatic disease.

The term "convalescence" relates to the period between the end of a disease and the restoration of complete health of a fish.

"Lipid(s)": The term lipid includes the common terms oil and fat, and includes all commonly known lipophilic substances such as mono-, di- and triglycerides, fatty acids, phospholipids, sterolesters such as cholesterol, shingolipids, etc.

Proteins are (macro)molecules consisting of sequences of amino acids linked though peptide bounds.

The term "processed protein" in the context of the present invention comprises:
- proteins with an increased degree of hydrolysation compared to untreated proteins, i.e. the original protein is cut into smaller pieces, i.e. peptides. These proteins or peptides may have a higher mount of oligopeptides including tri- and dipetides, as well as free amino acids
- partly denaturated proteins
- water-soluble proteins and/or
- proteineous compounds with an increased digestibility

Digestibility: Property to readily and easily digest ingested feed compounds in the intestinal tract into compounds suitable for absorption and subsequent assimilation.

Absorption: Passive and/or active uptake of (digested) feed compounds by the gut epithelium.
Appetite: desire for food resulting in an increased feed intake by the fish
Biomass: total amount of biomass is either the number of fish multiplied with the weight, or fish population in defined location such as a cage.

"Risk for a Pancreas disease infection": A risk for a pancreatic disease infection is present when the fish has an increased change of exposure to a PD infectious virus, such as the SAV virus, e.g. if there is a disease in the same aquaculture site or in a aquaculture site in a distance which make it possible that the fish could be infected, during transport of fish or infected material between aquaculture sites, due to infected wild fish, the SAV-virus is detected in the fish population but with no clinical symptoms, or due to environmental risk conditions such as temperature.

## Claims

1. Feed composition comprising conventional feed ingredients such as protein, lipid, ash, vitamins, minerals, carbohydrates, pigments, wherein the relation of protein to lipid is more than 1, based on weight, for use in alleviation, faster recovery and/or treatment of salmonids with Pancreas disease, wherein the feed composition is provided from the registration of the first symptoms of the infection in fish or a fish population or from the detection of SAV virus in the population.

2. Feed composition accordance with claim 1, wherein the relation of protein to lipid is in the range of 1 to 3, based on the weight.

3. Feed composition in accordance with claim 1, wherein the relation of protein to lipid is in the range of 1.5 to 2.5, based on weight, preferable about 2 based on weight.

4. Feed composition in accordance with claim 1, wherein the amount of protein in relation to the total weight of the feed composition is in the range of 30%to 70%, more preferable about 40% to 60%, and more preferable about 45% to 55%.

5. Feed composition in accordance with one of the preceding claims, wherein the feed composition further comprises an immune stimulating and/or anti-inflammation compound.

6. Feed composition in accordance with one of the preceding claims, wherein said feed composition further comprises semiessential nutrients such as nucleotides, nucleosides or both.

## Patentansprüche

1. Futterzusammensetzung, die übliche Futterbestandteile wie Protein, Lipid, Asche, Vitamine, Mineralien, Kohlenhydrate, Pigmente umfasst, wobei das Verhältnis von Protein zu Lipid gewichtsbasiert größer als 1 ist, zur Verwendung bei der Linderung, schnelleren Gesundung und/oder der Behandlung von Salmoniden bei einer Pankreaserkrankung, wobei die Futterzusammensetzung ab der Registrierung der ersten Symptome der Infektion bei Fisch oder einer Fischpopulation oder ab der Erfassung des Lachs-Alphavirus (SAV) in der Population bereitgestellt wird.

2. Futterzusammensetzung nach Anspruch 1, wobei das Verhältnis von Protein zu Lipid gewichtsbasiert im Bereich von 1 bis 3 liegt.

3. Futterzusammensetzung nach Anspruch 1, wobei das Verhältnis von Protein zu Lipid gewichtsbasiert im Bereich von 1,5 bis 2,5 liegt, vorzugsweise bei etwa 2 liegt.

4. Futterzusammensetzung nach Anspruch 1, wobei die Menge an Protein im Verhältnis zum Gesamtgewicht der Futterzusammensetzung im Bereich von 30% bis 70%, bevorzugt bei etwa 40% bis 60%, und noch bevorzugter bei etwa 45% bis 55% liegt.

5. Futterzusammensetzung nach einem der vorhergehenden Ansprüche, wobei die Futterzusammensetzung weiter eine immunstimulierende und/oder entzündungshemmende Zusammensetzung enthält.

6. Futterzusammensetzung nach einem der vorhergehenden Ansprüche, wobei die Futterzusammensetzung weiter semiessentielle Nährstoffe wie Nukleotide, Nukleoside oder beide umfasst.

## Revendications

1. Composition alimentaire comprenant les ingrédients alimentaires conventionnels comme des protéines, des lipides, des cendres, des vitamines, des minéraux, des glucides, des pigments, dans laquelle la proportion de protéines par rapport aux lipides est supérieure à 1, sur la base du poids, destinée à l'atténuation, l'accélération de la récupération et/ou le traitement de salmonidés affectés par une maladie du pancréas, **caractérisée en ce que** la composition alimentaire est prévue dès l'enregistrement des premiers symptômes de l'infection chez un poisson ou une population de poissons ou dès la détection du virus SAV dans la population.

2. Composition alimentaire selon la revendication 1, **caractérisée en ce que** la proportion de protéines par rapport aux lipides est comprise entre 1 et 3, sur la base du poids.

3. Composition alimentaire selon la revendication 1, **caractérisée en ce que** la proportion de protéines par rapport aux lipides est comprise entre 1,5 et 2,5 sur la base du poids, de préférence autour de 2 sur la base du poids.

4. Composition alimentaire selon la revendication 1, **caractérisée en ce que** la quantité de protéines par rapport au poids total de la composition alimentaire est compris entre 30% et 70%, de préférence entre 40% et 60%, et de préférence encore entre 45% et 55%.

5. Composition alimentaire selon l'une quelconque des revendications précédentes, **caractérisée en ce que** la composition alimentaire comprend en outre un composé stimulant de l'immunité et/ou anti-inflammatoire.

6. Composition alimentaire selon l'une quelconque des revendications précédentes, **caractérisée en ce que** la dite composition alimentaire comprend en outre des nutriments semi-essentiels tels des nucléotides, des nucléosides ou les deux.
